# EUROPEAN PATENT SPECIFICATION

(11) **EP 1 214 894 B1**
(45) Date of publication and mention of the grant of the patent: **17.09.2008**
(21) Application number: 01129422.0
(22) Date of filing: 10.12.2001
(51) Int. Cl.: A23L 1/304, A23L 1/30, A23L 2/52, A61K 33/06, A61K 33/10, A61K 35/56

(54) **Food formulations with a high calcium content**
Lebensmittelformulierungen mit einem hohen Kalziumgehalt
Compositions alimentaires riches en calcium

(30) Priority: 12.12.2000 IT MI002666
(43) Date of publication of application: 19.06.2002
(73) Proprietor: Rothlin, Ursula Mariah, 00192 Roma (IT)
(72) Inventor: Rothlin, Ursula Mariah, 00192 Roma (IT)
(74) Representative: Zeitler, Giselher

(56) References cited:
- EP-A- 0 790 004
- EP-A- 0 797 930
- EP-A- 1 008 303
- US-A- 4 540 584
- US-B1- 6 171 622
- DATABASE BIOSIS [Online] BIOSCIENCES INFORMATION SERVICE, PHILADELPHIA, PA, US; October 1999 (1999-10) ISHITANI KUNIHIKO ET AL: "Calcium absorption from the ingestion of coral-derived calcium by humans." Database accession no. PREV200000099114 XP002197113 & JOURNAL OF NUTRITIONAL SCIENCE AND VITAMINOLOGY, vol. 45, no. 5, October 1999 (1999-10), pages 509-517, ISSN: 0301-4800
- DATABASE WPI Section Ch, Week 199743 Derwent Publications Ltd., London, GB; Class D12, AN 1997-458013 XP002197114 & CN 1 117 821 A (NANHAI MARINE INST CHINESE ACAD SCI), 6 March 1996 (1996-03-06)
- DATABASE WPI Section Ch, Week 200043 Derwent Publications Ltd., London, GB; Class B04, AN 2000-483224 XP002197115 & CN 1 250 661 A (BAO Y), 19 April 2000 (2000-04-19)
- DATABASE WPI Section Ch, Week 199536 Derwent Publications Ltd., London, GB; Class B04, AN 1995-271420 XP002197116 & JP 07 170939 A (YAMAKAWA A), 11 July 1995 (1995-07-11)
- DATABASE WPI Section Ch, Week 199813 Derwent Publications Ltd., London, GB; Class D13, AN 1998-138204 XP002197117 & JP 10 014535 A (KARUNA KK), 20 January 1998 (1998-01-20)

## Description

The present invention refers to food formulations with a high calcium content.

In particular the invention relates to food formulations that can be used for prevention and as adjuvants in treatment and in prevention, especially in osteopathy (e.g. in the treatment of osteoporosis, arthrosis etc.).

Normally Ca is ingested along with milk and its by-products. Its absorption is influenced by ionisation and by the presence of vitamin D, parathyroid hormone and calcitonin, but is significantly reduced by the dietary presence of phytates and vegetable oxalates, fats and fibres. Lactose and some amino acids, such as lysin and arginine can by contrast increase calcium absorption.

Mg is present in many vegetables, but is barely absorbed by the gastrointestinal tract and is quickly eliminated by the kidneys.

The balances between these elements seem much more delicate in women and vary with age, as a result of biological, hormonal and dietary variations, but also due to diseases.

Many recent studies have clearly documented that an increased intake of Ca and Mg and a reduction of Na reduces the risk of death for cardiovascular diseases.

A correct calcium intake in children is essential for regular skeleton formation, but also during puberty and young adulthood.

In particular, it has been noted that the intake of calcium and magnesium in a diet is normally deficient. This is particularly true in low-calorie and low-fat diets that exclude the intake of milk and cheese or in cases of food intolerance to milk.

The required intake also increases during sports activities, especially in long-distance races such as marathons, cycle races etc., in which the body consumes larger amounts of calcium and magnesium. It should be noted that both these elements play a fundamental role in muscle performance. As a result an increased intake of these substances has proven useful in a variety of sports and leisure activities (such aerobics, bodybuilding, tennis, athletics and dancing).

A sufficient intake of Ca and Mg is important at special times in a woman's life, such as during pregnancy (to prevent eclampsia) and during breastfeeding. However, the oestrogen status and menopause are two situations, in which a lack of these of two minerals may lead to serious bone diseases, such as osteoporosis and fractures. Even in this case the risk is strictly associated with a reduced calcium intake. A lower intake of Mg reduces the calcium level and magnesium level in blood but promotes calcium deposition in the bones. This proves how important it is to strike the right balance between these two elements. In any case the relationship between a lack of calcium and osteoporosis has been frequently proven in elderly subjects.

To hinder or avoid the emergence of these problems, the calcium level in the bones must be restored by ingesting products very rich in calcium.

However, we know that living organisms can fix calcium only in ionic form easily in the bones. By contrast some foods may be rich in calcium, but their calcium content is bound in compounds that cannot ionise easily and are thus only able to provide a moderate calcium intake that can fix in the bones.

The foods currently available in the market that have a high calcium content all contain calcium compounds that cannot ionise easily (and thus have relatively unusable calcium) and, as a result can supply bones with a limited calcium intake.

Indeed traditional products comprise synthetic calcium that is barely absorbed and is thus not very bio-available, terrestrial mineral calcium, vegetable calcium, which cannot be absorbed due to phytates and oxalates, and animal calcium. The last-named substance is not very available in part because it always includes absorption inhibitors such as lipids.

To this we should also add that, for the calcium contained in traditional foods to fix in the bones, the body needs to contain other elements such as parathyroid hormone and calcitonin and, above all, vitamin D.

Therefore, the technical goal that the present invention sets out to achieve is to produce food formulations with a high calcium content that can very efficiently restore the calcium level in the bones and thus hinder decalcification.

In pursuit of this technical goal, an object of the invention is to produce formulations, which help significantly or minimise the intervention of other elements, particularly of vitamin D, to fix calcium in the bones.

The technical task as well as these and other objectives according to the present invention are achieved by producing food formulations with a high calcium content, characterised in that said calcium is contained in a product combined with other elements to form a compound that can provide a large amount of calcium in ionic form and in that they can be used prevalently in osteopathy.

Other characteristics of the present invention are also defined in the claims attached.

Further characteristics and advantages of the invention will be made clearer by the description of formulations that can be used in nutrition to help the body absorb large amounts of calcium besides the amount consumed with food.

According to the invention food formulations with a high calcium content comprise a product can provide a large amount of calcium ions and can be used in osteopathy.

In particular a basic constituent of the product comprises marine minerals and especially coral powder, in a weight percentage comprised between 20% and 50% of the formulation and preferably comprised between 33% and 41%.

Coral powder in turn contains calcium carbonate in weight percentage no less than 80% and preferably between 85% and 95%.

Coral powder includes high percentages of calcium carbonate, which *per se* cannot easily ionise and can thus scarcely be absorbed. However, surprisingly coral powder can provide an organism with very large amounts of ionised calcium that can easily fix in the bones, although the main ingredient consists of calcium carbonate.

Coral powder also contains magnesium in a weight percentage less than 15% and preferably between 5% and 10%. This level promotes calcium absorption and fixation in the bones.

Coral powder contains scleractinia powder (special type of coral), which consist of around 92% calcium carbonate and 7.6% magnesium.

It is an object of the present invention providing calcium food formulations, characterized in that they comprise scleractinia powder in a weight percentage ranging between 20% and 50% of the formulation, said scleractinia powder consisting of around 92% calcium carbonate and 7.6% magnesium.
In a preferred realisation, scleractinia powder is present in a weight percentage ranging between 33% and 41% of the formulation.

Coral containing compositions have been disclosed in the following documents: J.Nutr.Sci.Vitaminol (Tokyo) 1999 Oct; 45(5): 509-17; CN 1117821; CN 1250661; JP 7170939; EP A 0797930; US A 4540584; EP A 0790004; US B1 6171622; EP A 1008303; JP 10014535.
None of them discloses a calcium food composition comprising schleractinia powder to provide a composition for promoting calcium absorption and fixation into the bones.

Moreover, the formulations include an added ingredient to make them more appealing to consumers.

This ingredient added to enhance the taste of the formulations consists of, in a first example, sweeteners such as glucose or fructose or cyclamates.

Glucose constitutes a percentage between 30% and 90% of the formulation and preferably between 53.0 and 63.0%.

Although glucose is preferably used to make the formulations tastier for people, it also has applications in veterinary medicine (e.g. to make the formulations more appealing to horses according to the invention).

In a second example the ingredient added to enhance the taste of the formulations comprises meat extracts in a weight percentage less than 70% of the product w and preferably between 35.0 and 44.0% of the product.

Meat extracts are usually accompanied by mineral salts in weight percentage less than 50.0% of the formulation and preferably comprised between 15.0 and 25.0%.

Meat extracts and mineral salts are chiefly used in veterinary medicine.

Moreover the formulations also include an emulsifier in a weight percentage less than 5% of the formulation itself and preferably comprised between 2.5% and 3.0%. For instance it may consists of *colidon* K-25 and a binder in weight percentage less than 5% and preferably comprised between 1.8% and 2.2% of the formulation, e.g. composed of lecithin 95%.

The formulations in various practical examples also contain organic sulphur in weight percentage less than 30% of the product and preferably less than 15%.

Organic sulphur is identical to elements containing sulphur already present in the body and proves very useful as it has analgesic, energetic and anti-ageing properties and helps, as an improvement, the physical and psychophysical condition of its consumers.

According to the finding the formulations, in other examples, include amino acids in a weight percentage less than 3% and preferably comprised between 0.5 and 1.1% and/or proteins in weight percentage less than 3% and preferably comprised between 0.5 and 1.1%.

Amino acids are used to promote calcium fixation and to increase its bio-availability. By contrast, proteins are used to enable the body itself to produce amino acids with the function outlined above.

In further examples, according to the invention the formulations also include extracts of officinal plants in weight percentage less than 20%.

Owing to their special properties, medicinal plants help to sustain and improve the psychophysical condition of those who ingest the formulations according to the finding.

According to the finding, the formulations containing glucose (or in any case a sweetener) are advantageously produced as a powder or as granules to be added to water or other liquid or semiliquid foodstuffs.

According to the finding the formulations containing meat extracts and mineral salts are by contrast produced as bars or in bulk.

Hereafter some examples of products according to the finding are described.

### Example 1:

In a first example according to the invention the formulation comprises:
- Coral powder:
   scleractinia, constitutes 36.6% in weight percentage of the product,
- Ingredient added to enhance the taste of (or sweeten) the formulation:
   Monohydric glucose constitutes 58.6% in weight percentage of the product,
- Emulsifier:
   *Colidon* K-25 constitutes 2.8% in weight percentage of the product,
- Binder:
   lecithin 95% constitutes 2.0% in weight percentage of the product,

This formulation particularly appeals both to persons and some animals (such as horses) and can be used as a sweetener and dietary supplement.

This formulation has proven very effective as it can provide the body with calcium in ionic form that is fixed in the bones in large amounts (nearly all calcium that the product contains is transferred in ionic form to the body).

### Example 2:

In a second example according to the invention the formulation comprises:
- Coral powder:
   scleractinia constitutes 36.6% in weight percentage of the product,
- Ingredient added to enhance the taste of the formulation:
   meat extracts constitutes 38.6% in weight percentage of the product,
   mineral salts constitutes 20.0% in weight percentage of the product,
- Emulsifier:
   *Colidon* K-25 constitutes 2.8% in weight percentage of the product,
- Binder:
   Lecithin 95% constitutes 2.0% in weight percentage of the product,

The formulation of example 2 shares similar characteristics with formulation 1 but is especially developed for veterinary medicine.

### Example 3:

In a third example according to the invention the formulation comprises:
- Coral powder:
   scleractinia constitutes 36.6% in weight percentage of the product,
- Ingredient added to enhance the taste of (or sweeten) the formulation:
   Monohydric glucose constitutes 47.6% in weight percentage of the product,
- Emulsifier:
   *Colidon* K-25 constitutes 2.8% in weight percentage of the product,
- Binder:
   Lecithin 95% constitutes 2.0% in weight percentage of the product,
- Organic sulphur constitutes 11.0% in weight percentage of the product,

Besides the characteristics of the formulation in example 1, this product sustains the consumer's physical and psychophysical condition.

### Example 4:

In a fourth example according to the invention the formulation comprises:
- Coral powder:
   Scleractinia constitutes 36.6% in weight percentage of the product,
- Ingredient added to enhance the taste (sweeten) of the formulation:
   Monohydric glucose, constitute 57.7% in weight percentage of the product,
- Emulsifier:
   *Colidon* K-25 constitutes 2.8% in weight percentage of the product,
- Binder:
   Lecithin 95% constitutes 2.0% in weight percentage of the product,
- Proteins constitute 1.0% in weight percentage of the product,

Besides the characteristics of the formulation in example 1, this formulation contains proteins that promote calcium fixation and increase its bio-availability.

### Example 5:

In a fifth example according to the invention the formulation comprises:
- Coral powder:
   scleractinia constitutes 36.6% in weight percentage of the product,
- Ingredient added to enhance the taste (sweeten) of the Formulation:
   Monohydric glucose constitutes 57.6% in weight percentage of the product,
- Emulsifier:
   *Colidon* K-25 constitutes 2.8% in weight percentage of the product,
- Binder:
   lecithin 95% constitutes 2.0% in weight percentage of the product,
   Amino acids:
   phenylalanine constitutes 1.0 % in weight percentage of the product,

Besides the product characteristics given in example 1, this product promotes greater concentration, serves to control one's appetite and has a tonic and stimulating effect.

### Example 6:

In a sixth example, according to the invention the product comprises:
- Coral powder:
   Scleractinia constitutes 36.6% in weight percentage of the product,
- Ingredient added to enhance the taste (sweeten) of the formulation:
   Monohydric glucose constitutes 38.6% in weight percentage of the product,
- Emulsifier:
   *Colidon* K-25 constitutes 2.8% in weight percentage of the product,
- Binder:
   Lecithin 95% constitutes 2.0% in weight percentage of the product,
- Extracts of medicinal plants:
   Burdock root (*arctium lappa*) constitutes 4.6% in weight percentage of the product,
   *Silybum marianum* (milk thistle) constitutes 9.2% in weight percentage of the product,
   Gentian (gentiana lutea) constitutes 3.0% in weight percentage of the product,
   *Vitis vinifera* grapes constitute 1.6% in weight percentage of the product,
   Butcher's broom (ruscus aculeatus) constitutes 1.6% in weight percentage of the product.

Besides the characteristics of the formulation given in example 1, this formulation encourages blood circulation and supply to organs and peripheral body tissues.

Furthermore, in other examples, the product containing coral powder includes low-calorie diet lines of the nourishment of elderly persons, children, sports enthusiasts, pregnant women etc.

Indeed in these cases the need to respect the recommended calcium intake plays a very urgent role.

Coral powder is not soluble and, therefore, it is best added to other powders used for nutritional purposes that may form solids or suspensions.

The concentration of the coral powder in low-calorie diet lines is less than 1% and preferably ranging from 0.2 to 0.4 %. For instance, it has the following values: pasta 0.2%, biscuits and cookies 0.3%, crackers 0.3%, yoghurt 0.3%, powder chocolate 0.4%, mashed potatoes 0.2%, diet bars made from chocolate 0.4%, cocoa 0.4%.

The product containing coral powder has the benefit of including breakfast foods as a whole such as cornflakes, French toast, croissants and fresh bread.

In practice we have observed how, according to the invention, the food formulations with a high calcium content are especially beneficial because serve to restore calcium in the bones very efficiently and also reduce the need for vitamin D supplements.

It has the beneficial effect that people who ingest calcium no longer need to expose themselves to sunlight.

Accordingly, it is a further object of the present invention the use of the high calcium containing food formulations according to the above description, for the preparation of pharmaceutical compositions for providing an amount of calcium in ionic form to promote calcium absorption and fixation into the bones.

Food formulations with a high calcium content as described above are susceptible to a plethora of alterations and variants, all of which constitute the inventive concept. Moreover all the details may be replaced by technically equivalent elements.

In practice any materials or dimensions may be used depending on requirements and technological development.

## Claims

1. Calcium food formulations, **characterized in that** they comprise scleractinia powder in a weight percentage ranging between 20% and 50% of the formulation, said scleractinia powder consisting of around 92% calcium carbonate and 7.6% magnesium.

2. Formulations according to claim 1, in which said scleractinia powder is present in a weight percentage ranging between 33% and 41% of the formulation.

3. Formulations according to claims 1 and 2, further comprising sweeteners such as glucose, fructose or cyclamates.

4. Formulations according to claim 3, in which said glucose is contained in a weight percentage comprised between 30% and 63% of the formulation.

5. Formulations according to anyone of the foregoing claims, further comprising meat extracts in a weight percentage lower than 70% of the formulation, preferably comprised between 35.0% and 44.0%.

6. Formulations according to claim 5, further comprising mineral salts in a weight percentage lower than 50% of the formulation, preferably comprised between 15.0% and 25.0%.

7. Formulations according to anyone of the foregoing claims, further comprising an emulsifier in a weight percentage lower than 5% of the formulation, preferably comprised between 2.5% and 3.0%.

8. Formulations according to claim 7, in which said emulsifier is colindon K-25.

9. Formulations according to anyone of the foregoing claims, further comprising a binder in a weight percentage lower than 5% of the formulation, preferably comprised between 1.8% and 2.2%.

10. Formulations according to claim 9, in which said binder is lecithin 95%.

11. Formulations according to anyone of the foregoing claims, further comprising organic sulphur in a weight percentage lower than 30% of the formulation, preferably less than 15%.

12. Formulations according to anyone of the foregoing claims, further comprising amino acids in a weight percentage lower than 3% of the formulation, preferably comprised between 0.5% and 1.1%; and/or proteins in a weight percentage lower than 3% of the formulation, preferably comprised between 0.5% and 1.1%.

13. Formulations according to anyone of the foregoing claims, further comprising extracts of officinal plants in a weight percentage lower than 20% of the formulation.

14. Use of one of the formulations according to anyone of claims 1 to 13, for the preparation of pharmaceutical compositions for providing an amount of calcium in ionic form to promote calcium absorption and fixation into the bones.

## Patentansprüche

1. Mit Calcium angereicherte Nahrungsmittelformulierungen, die **dadurch gekennzeichnet sind, dass** sie zwischen 20 und 50 Gewichtsprozenten Scleractinia-Pulver [Steinkoralle-Pulver] enthalten, wobei besagtes Scleractinia-Pulver aus ca. 92 % Calciumkarbonat und 7,6 % Magnesium besteht.

2. Formulierungen gemäss Patentanspruch 1, deren Gewichtsanteil an besagtem Scleractinia-Pulver zwischen 33 und 41 % der Formulierung beträgt.

3. Formulierungen gemäss den Patentansprüchen 1 und 2, die ferner Süssstoffe wie Glukose, Fruktose oder Zyklamate, enthalten.

4. Formulierungen gemäss Patentanspruch 3, die einen Gewichtsanteil von 30 bis 63 % besagter Glukose enthalten.

5. Formulierungen gemäss einem der vorangehenden Patentansprüche, die ausserdem Fleischextrakte mit einem Gewichtsanteil von weniger als 70 % der Formulierung, vorzugsweise zwischen 35 und 44 %, enthalten.

6. Formulierungen gemäss Patentanspruch 5, die ferner Mineralsalze mit einem Gewichtsanteil von weniger als 50 % der Formulierung, vorzugsweise zwischen 15 und 25 %, enthalten

7. Formulierungen gemäss einem der vorangehenden Patentansprüche, die ausserdem einen Emulgator mit einem Gewichtsanteil von weniger als 5 % der Formulierung, vorzugsweise zwischen 2,5 und 3,0 %, enthalten

8. Formulierungen gemäss Patentanspruch 7, bei denen besagter Emulgator Colidon K-25 ist.

9. Formulierungen gemäss einem der vorangehenden Patentansprüche, die ferner ein Bindemittel mit einem Gewichtsanteil von weniger als 5 % der Formulierung, vorzugsweise zwischen 1,8 und 2,2 %, enthalten.

10. Formulierungen gemäss Patentanspruch 9, bei denen besagtes Bindemittel 95 % Lezithin ist.

11. Formulierungen gemäss einem der vorangehenden Patentansprüche, die ferner organischen Schwefel mit einem Gewichtsanteil von weniger als 30 % der Formulierung, vorzugsweise von weniger als 15 %, enthalten.

12. Formulierungen gemäss einem der vorangehenden Patentansprüche, die ferner Aminosäuren in einem Gewichtsanteil von weniger als 3 % der Formulierung, vorzugsweise zwischen 0,5 und 1,1 %, enthalten; und/oder Proteine mit einem Gewichtsanteil von weniger als 3 % der Formulierung, vorzugsweise zwischen 0,5 und 1,1 %.

13. Formulierungen gemäss einem der vorangehenden Patentansprüche, die ferner Extrakte von Arzneipflanzen mit einem Gewichtsanteil von weniger als 20 % der Formulierung enthalten.

14. Verwendung einer der Formulierungen gemäss einem der Patentansprüche 1 bis 13 für die Zubereitung von pharmazeutischen Verbindungen für die Zufuhr von ionischem Calcium zur Förderung der Aufnahme und Bindung von Calcium in den Knochen.

## Revendications

1. Formulations alimentaires contenant du calcium, **caractérisées en ce qu'**elles comprennent de la poudre de scleractinia selon un pourcentage pondéral compris dans la plage de 20 % à 50 % de la formulation, ladite poudre de scleractinia consistant en environ 92 % de carbonate de calcium et 7,6 % de magnésium.

2. Formulations selon la revendication 1, dans lesquelles ladite poudre de scleractinia est présente selon un pourcentage pondéral compris dans la plage de 33 % à 41 % de la formulation.

3. Formulations selon les revendications 1 et 2, qui comprennent en outre des édulcorants tels que le glucose, le fructose ou les cyclamates.

4. Formulations selon la revendication 3, dans lesquelles ledit glucose est présent selon un pourcentage pondéral compris entre 30 % et 63 % de la formulation.

5. Formulations selon l'une quelconque des revendications précédentes, qui comprennent en outre des extraits de viande selon un pourcentage pondéral inférieur à 70 % de la formulation, de préférence compris entre 35,0 % et 44,0 %.

6. Formulations selon la revendication 5, qui comprennent en outre des sels minéraux selon un pourcentage pondéral inférieur à 50 % de la formulation, de préférence compris entre 15,0 % et 25,0 %.

7. Formulations selon l'une quelconque des revendications précédentes, qui comprennent en outre un émulsifiant selon un pourcentage pondéral inférieur à 5 % de la formulation, de préférence compris entre 2,5 % et 3,0 %.

8. Formulations selon la revendication 7, dans lesquelles ledit émulsifiant est le colidon K-25.

9. Formulations selon l'une quelconque des revendications précédentes, qui comprennent en outre un liant selon un pourcentage pondéral inférieur à 5 % de la formulation, de préférence compris entre 1,8 % et 2,2 %.

10. Formulations selon la revendication 9, dans lesquelles ledit liant est la lécithine à 95 %.

11. Formulations selon l'une quelconque des revendications précédentes, qui comprennent en outre du soufre organique selon un pourcentage pondéral inférieur à 30 % de la formulation, de préférence inférieur à 15 %.

12. Formulations selon l'une quelconque des revendications précédentes, qui comprennent en outre des acides aminés selon un pourcentage pondéral inférieur à 3 % de la formulation, de préférence compris entre 0,5 % et 1,1 % ; et/ou des protéines selon un pourcentage pondéral inférieur à 3 % de la formulation, de préférence compris entre 0,5 % et 1,1 %.

13. Formulations selon l'une quelconque des revendications précédentes, qui comprennent en outre des extraits de plantes officinales selon un pourcentage pondéral inférieur à 20 % de la formulation.

14. Utilisation de l'une des formulations selon l'une quelconque des revendications 1 à 13 pour préparer des compositions pharmaceutiques destinées à fournir une quantité de calcium sous forme ionique dans le but de favoriser l'absorption du calcium et sa fixation par les os.
